# EUROPEAN PATENT APPLICATION

(11) **EP 2 033 660 A1**
(43) Date of publication of application: **11.03.2009**
(21) Application number: 07017412.3
(22) Date of filing: 05.09.2007
(51) Int. Cl.: A61K 47/48, A61P 29/00, A61P 17/06, A61P 11/06, A61P 19/02, A61P 37/06, A61P 37/00

(54) **Functionalized nanoparticles for the inhibition of selectin-mediated cell adhesion**

(71) Applicant: Freie Universität Berlin, 14195 Berlin (DE); Charité-Universitätsmedizin Berlin, 10117 Berlin (DE)
(72) Inventor: Reissig, Hans-Urlich, 12165 Berlin (DE); Dernedde, Jens, 10629 Berlin (DE); Schlecht, Sabine, 10963 Berlin (DE); Roskamp, Meike, 10717 Berlin (DE)
(74) Representative: Engelhard, Markus

(57) **Abstract**

The present invention relates to functionalized nanoparticles inhibiting selectin-mediated cell adhesion and their use as anti-inflammatory therapeutics.

## Description

The present invention relates to functionalized nanoparticles inhibiting selectin-mediated cell adhesion and their use as anti-inflammatory therapeutics.

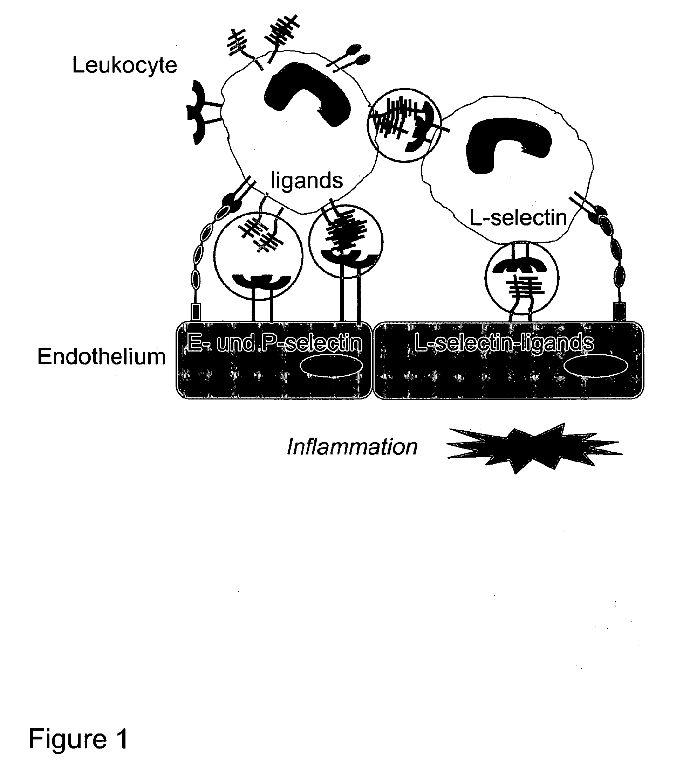

Recruitment of white blood cells (leukocytes) to the endothelial cells lining blood vessels and their subsequent emigration into the adjoining tissue are observed in all forms of the inflammatory response (see Figure 1). Initial contact and rolling along the endothelial surface is mediated by transient receptor-ligand interactions between the three selectins and their ligands (Ley K., Trends Mol. Med. 9 (2003) 2638). Tight contact between the leukocytes and the endothelium is subsequently achieved by the interaction of activated integrins with adhesion molecules of the immunoglobulin superfamily (Springer T.A., Nature 346 (1990) 425-434). In addition to desirable defensive effects and repair of tissue defects the uncontrolled emigration of leukocytes from the bloodstream can also be of pathological importance and may lead to tissue damage (Lefer D.J., Annu. Rev. Pharmacol. Toxicol. 40 (2000) 283-294). The general involvement of endothelial cell adhesion molecules in acute and chronic inflammatory processes thus makes them to suitable targets for diagnosis and therapy (Boehncke W.H. et al., Exp. Dermatol. 14 (2005) 70-80).

E- and P-selectin as well as ligands of L-selectin are inflammatory-dependently expressed on the microvascular endothelium, whereas L-selectin is presented by leukocytes (Ley K., Trends Mol. Med. 9 (2003) 2638; Springer T.A., Nature 346 (1990) 425-434). Findings that the tetrasaccharide Sialyl Lewis X (sLex) is a crucial binding partner of selectins and that polyvalency is a key for the targeted blockade of leukocyte adhesion are well-established and have formed the basis for the development of various selectin inhibitors (Simanek et al., Chem. Rev. 98 (1998) 833-862). However, targeting selectin has not yet lead to the development of market-ready therapeutics despite the fact that high-affinity inhibitors are at hand (Simanek et al., Chem. Rev. 98 (1998) 833-862).

Currently, therapeutic intervention in the case of rheumatoid arthritis is achieved by the use of inhibitors of the inflammatory cytokine TNFα (Infliximab, Etanercept). The anti-integrin antibody Efalizumab is on the market and approved for the systemic therapy in the case of psoriasis. Other substances are currently tested in clinical trials, such as the substance Bimosiamose (Revotar, Henningsdorf), a pan-selectin antagonist, which belongs to the class of small molecule drugs and is supposed to be used for asthma, psoriasis, reperfusion damages.

Linear neoglycopolymers carrying sulfated sLex structures as well as sulfated dendritic polyethylene oxide (PEO) glycopolymers have been described before and can achieve IC₅₀ values in the lower nanomolar range (Simanek et al., Chem. Rev. 98 (1998) 833-862; Mowery et al., Chem. Biol. 11 (2004) 725-732; Rele et al., J. Am. Chem. Soc. 127 (2005) 10132-10133). Functionalized nanoparticles, in particular glyco-nanoparticles with a core of polymer or gold, have so far been mainly used with respect to diagnostic and imaging applications. Their potential therapeutic application is currently being tested using animal models (John et al., FASEB J. 17 (2003) 2296-2298; Rojo et al., ChemBioChem 5 (2004) 291-297). Various carbohydrate derivatives or carbohydrate mimetics analogous to sLex structures have been tested as ligands of selectin as well (Wong et al., Chem. Rev. 98 (1998) 833-863; Kaila et al., J. Med. Chem. 48 (2005) 4346-4357).

It was an object of the present invention to provide for a high-affinity inhibitor of selectin-ligand interactions, which is suitable for the treatment of diseases, particularly inflammatory diseases. It was another object of the present invention to provide for a high-affinity inhibitor that can be easily synthesized and is stable in a physiological environment.

The objects of the present invention are solved by a functionalized nanoparticle, comprising
a core,
a shell coating said core, formed by a monolayer of a linker molecule, and
at least one polar functional group covalently linked to said linker molecule.

In one embodiment said core is formed by gold.

In one embodiment said core has a diameter ≤ 50 nm, preferably ≤ 25 nm, more preferably ≤ 15 nm.

In one embodiment said linker molecule is a linear or branched, preferably a linear alkyl chain, with at least one binding functionality for the attachment of said linker molecule to said core.

In one embodiment said alkyl chain has 3 to 26 C-atoms, preferably 6 to 26 C-atoms, more preferably 10 to 26 C-atoms.

In one embodiment said binding functionality comprises at least one sulfur atom.

In one embodiment said binding functionality is selected from the group comprising thiol (SH), thiolate (S⁻) or disulfide (S₂).

In one embodiment said polar functional group is covalently linked to said linker molecule via a bond selected from the group comprising peptide bond, carbon-carbon bond, carbon-oxygen bond, carbon-sulfur bond.

In one embodiment said polar functional group is selected from the group comprising carboxyl (COOH), sulfate (OSO₃⁻), sulfonate (SO₃⁻), hydroxyl (OH), and a poly-hydroxylated heterocyclic compound.

In one embodiment said polar functional group is a polyhydroxylated heterocyclic compound.

In one embodiment said heterocyclic compound is an oxygen-containing heterocyclic compound.

In one embodiment said oxygen-containing heterocyclic compound is selected from the group comprising amino pyranes, amino oxepanes, amino oxacanes, and amino furan derivatives.

In one embodiment said polyhydroxylated heterocyclic compound is oxidized to carboxylic acid in primary hydroxylated positions.

In one embodiment said polyhydroxylated heterocyclic compound is sulfated.

In one embodiment said linker molecule is represented by the formula 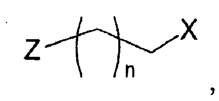 wherein
Z represents a binding functionality as defined above, wherein Z is selected from the group comprising SH, S⁻, and S₂,
n is not less than 2 and equal to or less than 25, and
X represents a polar functional group as defined above, wherein
X is selected from the group comprising COOH, OSO₃⁻, SO₃⁻, OH, and a polyhydroxylated heterocyclic compound, preferably an oxygen-containing heterocyclic compound being represented by the formula 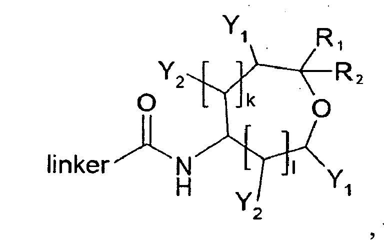 wherein
k is 0 or 1,
1 is 0, 1, or 2,
Y₁ is selected from the group comprising CH₂OH, COOH, and CH₂OSO₃⁻,
Y₂ is selected from the group comprising OH and OSO₃⁻,
and R₁ and R₂ are selected from the group comprising H, alkyl, aryl, and heteroaryl, preferably an alkyl, wherein said alkyl is selected from the group comprising C1-C12 alkyl, preferably methyl, ethyl, propyl, isopropyl, butyl, t-butyl, a perhalogenated alkyl, wherein the halogenide is selected from the group comprising F, Cl, Br, and I, preferably F, and a hydroxylated alkyl, preferably monohydroxylated alkyl.

The objects of the present invention are also solved by a method for producing a functionalized nanoparticle as defined above comprising the covalent linking of polar functional groups, preferably polyhydroxylated heterocyclic compounds as defined above, to linker molecules bound to a gold core.

The objects of the present invention are also solved by a nanoparticle as defined above for the treatment of diseases.

The objects of the present invention are also solved by the use of a nanoparticle as defined above for the production of a medicament for the treatment of inflammatory diseases.

In one embodiment said inflammatory diseases are chronic inflammatory diseases, in particular rheumatoid arthritis, asthma, and psoriasis.

In one embodiment said inflammatory diseases are ischemia reperfusion damages or graft repulsion.

The objects of the present invention are also solved by the use of a nanoparticle as defined above as selectin inhibitor.

In one embodiment the nanoparticle as defined above is used as inhibitor of L-selectin and/or P-selectin and/or E-selectin.

The term "functionalized nanoparticle" as used herein is meant to refer to a nanoparticle that is provided with functional groups or molecules that allow the binding to a specific target, such as a protein, preferably a specifically selected protein, such as a selectin, e.g. an L-, P-, or E-selectin, or a nanoparticle that is provided with functional groups or molecules that increase the stability of the nanoparticle in a given environment, e.g. by preventing aggregation.

The term "core" as used herein is meant to refer to a core or nucleus that provides a base for the attachment of linker molecules via functional groups, such as thiol groups.

The term "shell" as used herein is meant to refer to a shell covering the above-defined core and being formed by a monolayer, preferably a closed monolayer of a plurality of linker molecules. For example, in one embodiment of the present invention a core with a diameter of 6.2 nm is coated by a monolayer formed by approximately 1300 linker molecules.

The term "linker molecule" as used herein is meant to refer to a molecule, which is able to bind to the above-defined core via at least one terminal or non-terminal binding functionality, such as a thiol group, and which is provided with at least one additional polar functional group, that allows for binding to a target protein, such as selectin.

The term "polyhydroxylated" as used herein is meant to refer to the introduction of a plurality of hydroxyl groups, which are connected to the heterocyclic ring or to side chain carbons.

The term "... is sulfated" as used herein is meant to refer to the formation of a sulfate group from one to up to all free hydroxyl groups by the addition of a SO₃-complex.

The term "selectin inhibitor" as used herein is meant to refer to exogenous molecules or compounds, such as the nanoparticles described herein, that are able to bind to selectins with high affinity, thereby disrupting or preventing physiological selectin-ligand interactions.

The inventors have surprisingly found that functionalized nanoparticles according to the present invention are excellent inhibitors of selectin-ligand interactions. With half inhibitory concentrations (IC₅₀) in the lower picomolar range, they have a far higher affinity for selectins than any other known selectin inhibitor. By efficiently inhibiting selectin-ligand interactions, which in turn mediate the interaction between leukocytes and endothelial cells, the functionalized nanoparticles according to the present invention could provide a potent way of treating a variety of inflammatory diseases.

Nanoparticles according to the present invention, in particular when comprising a sulfated heterocyclic compound, preferably amino pyrane, show a high stability with respect to aggregation at physiological pH values, even at concentrations in the high nanomolar range. The covalent linking of polyhydroxylated or sulfated heterocyclic compounds to the linker molecules of the shell provides for hydrolytic stability. Furthermore, the fact that heterocyclic compounds, such as amino pyrane, are non-physiological carbohydrate mimetics should provide for metabolic stability.

Reference is now made to the figures, wherein
**Figure 1** is a schematic illustration of the interactions between endothelial and leukocyte adhesion molecules during leukocyte adhesion. Circles highlight selectin-ligand interactions.
**Figure 2** shows the syntheses of various functionalized nanoparticles. **Figure 2A** shows the synthesis of gold colloids covered by N-hydroxysuccinimid-11-mercaptoundecanoate, **Figure 2B** shows the synthesis of gold colloids functionalized with amino pyrane (AP), **Figure 2C** shows the sulfation of AP-functionalized gold colloids, **Figure 2D** shows the synthesis of gold colloids covered by 11-mercaptoundecanyl sulfate, and **Figure 2E** shows the synthesis of gold colloids covered by 11-mercaptoundecanoic acid.
**Figure 3** is a graph showing the high-affinity competitive inhibition of L-selectin-ligand interaction by functionalized gold colloids, as determined by surface plasmon resonance (SPR).
**Figure 4** is a graph showing the inhibition of L-selectin-ligand interaction depending on the degree of sulfation of the functional group amino pyrane (AP), as determined by surface plasmon resonance (SPR).
**Figure 5** is a graph showing the selectin-specific inhibitory effects of nanoparticles functionalized with 11-mercaptoundecanoic acid (MUDS), as determined by surface plasmon resonance (SPR).

The invention is now further described by reference to the following examples which are intended to illustrate, not to limit the scope of the invention.

### Example 1

### Production of functionalized colloidal gold nanoparticles

### A. Gold colloids covered by N-hydroxysuccinimid-11-mercaptoundecanoate

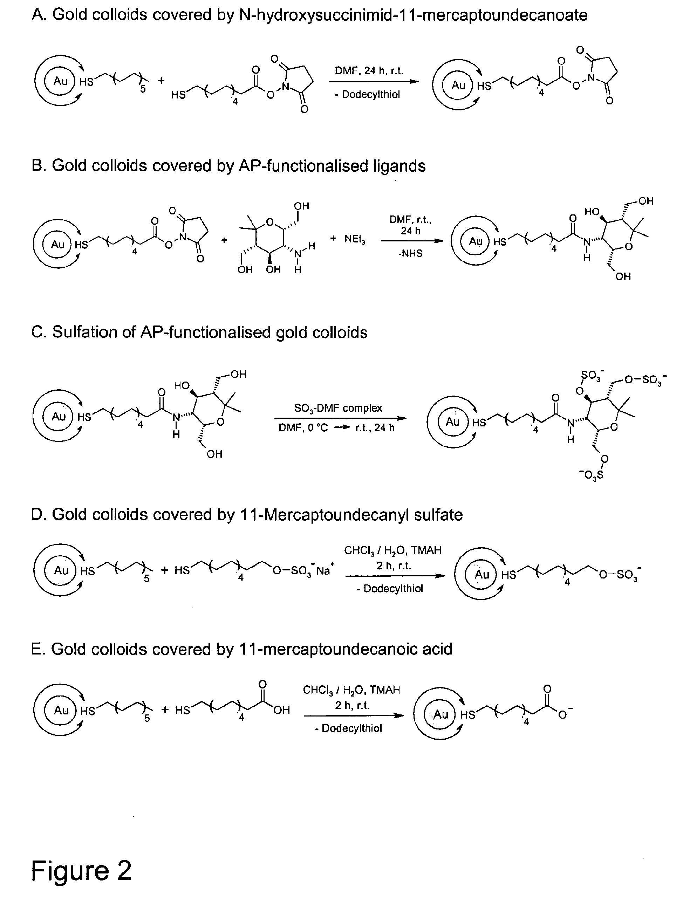

With stirring, a solution of dodecylthiol-covered gold colloids (6 nmol, d = 6.2 nm) in 4 ml of chloroform was dropped into a solution of N-hydroxysuccinimid-11-mercaptoundecanoate (0.189 g, 0.6 mmol, 10⁵ eq) in 40 ml of anhydrous DMF. After 10 minutes, the solution was concentrated to 30 ml and stirred at room temperature (r.t.) for another 24 h. Subsequently, the particle solution was dialysed against DMF (MWCO: 4000-6000, three times for 3 h with 200 ml of DMF) **(****Figure 2A****)**.

### B. Gold colloids functionalized with AP

Polyhydroxylated amino pyranes were prepared according to established protocols (Reissig et al., Angew. Chem. 117 (2005) 6383-6387; Reissig et al., Angew. Chem. Int. Ed. 44 (2005) 6227-6231); Yekta S. et al., Synlett (2007) 2069-2072). With stirring, a solution of the amino pyrane (AP) (0.02 mmol, 2·10⁴ eq) in 0.5 ml of anhydrous DMF was dropped into a solution of gold colloids covered by N-hydroxysuccinimid-11-mercaptoundecanoate (1 nmol NP, d = 6.2 nm, see above) in 6.6 ml of anhydrous DMF. The reaction mixture was stirred for 10 minutes before adding triethylamine (20 µl, 0.14 mmol, 35·10⁵ eq). After further 15 h of stirring at r.t. the nanoparticle solution was dialysed against DMF (MWCO: 4000-6000, three times for 3 h with 200 ml of DMF) **(****Figure 2B****)**.

### C. Sulfation of AP-functionalized gold colloids

At 0 °C and with stirring a solution of SO₃·DMF (15.3 mg, 0.1 mmol, 10⁵ eq) in 1 ml of anhydrous DMF was dropped slowly into a solution of amino pyrane-functionalized gold colloids (1 nmol, d = 6.2 nm) in 10 ml of anhydrous DMF. After 24 h of stirring at r.t. the nanoparticle solution was dialysed first against DMF (MWCO: 4000-6000, three times for 3 h with 200 ml of DMF), then against millipore water (MWCO: 4000-6000, three times for 3 h with 200 ml of H₂O) **(****Figure 2C****)**.

### D. Gold colloids covered by 11-mercaptoundecanyl sulfate

With stirring a solution of dodecylthiol-covered gold colloids (3 nmol, d = 6.2 nm) in 1 ml of chloroform was dropped into a solution of 11-mercaptoundecanyl sulfate sodium salt (29.8 mg, 0.1 mmol, 3.3·10⁴ eq) and tetramethylammonium hydroxide (TMAH) (50 µl, 0.14 mmol, 4.7·10⁴ eq) in 5 ml of millipore water. After 2 h of stirring a complete phase transfer had taken place. The phases were separated and the aqueous phase was dialysed against millipore water (MWCO: 4000-6000, three times for 3 h with 200 ml of H₂O) **(****Figure 2D****).**

### E. Gold colloids covered by 11-mercaptoundecanoic acid

With stirring a solution of dodecylthiol-covered gold colloids (3 nmol, d = 6.2 nm) in 1 ml of chloroform was dropped into a solution of 11-mercaptoundecanoic acid (44 mg, 0.2 mmol, 6.7·10⁴ eq) and TMAH (200 µl, 0.56 mmol, 18.8·10⁴ eq) in 5 ml of millipore water. After 2 h of stirring a complete phase transfer had taken place. The phases were separated and the aqueous phase was dialysed against millipore water (MWCO: 4000-6000, three times for 3 h with 200 ml of H₂O) **(****Figure 2E****)**.

### Example 2

### In vitro inhibition of selectin-ligand interaction using functionalized colloidal gold nanoparticles

Following functionalized colloidal gold (Au) nanoparticles (diameter = 6.2 nm) were tested in a competitive *in vitro* P-selectin-ligand binding assay in order to determine their half inhibitory concentrations (IC₅₀):
1. Nanoparticles functionalized with 11-mercaptoundecanoic acid (MUDS)
2. Nanoparticles functionalized with 11-mercaptoundecanyl sulfate (MUDSulfate)
3. Nanoparticles functionalized with 11-mercaptoundecanoic acid covalently linked to polyhydroxylated amino pyrane (AP)
4. Nanoparticles functionalized with 11-mercaptoundecanoic acid covalently linked to sulfated amino pyrane (sulf-AP)
Nanoparticles 1, 2, and 4 were tested in a competitive *in vitro* L-selectin-ligand binding assay.

Selectin-ligand binding was analyzed by surface plasmon resonance (SPR). First, the binding (detected as resonance units) of selectin-coated nanoparticles to a selectin ligand, immobilized on a sensor chip, was tested, and the resulting signal was henceforth referred to as 100% binding. Preincubation of the selectin-coated particles with varying concentrations of a selectin inhibitor decreased the binding signal. The calculated IC₅₀ value (half inhibitory concentration) is the molar concentration of the inhibitor needed to reduce the binding signal to 50% of the initial value.

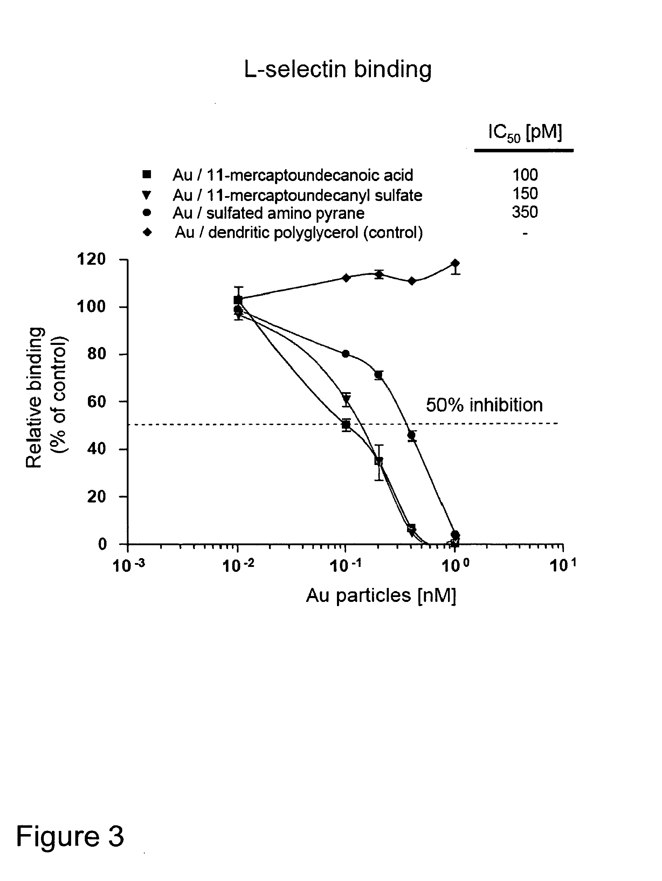

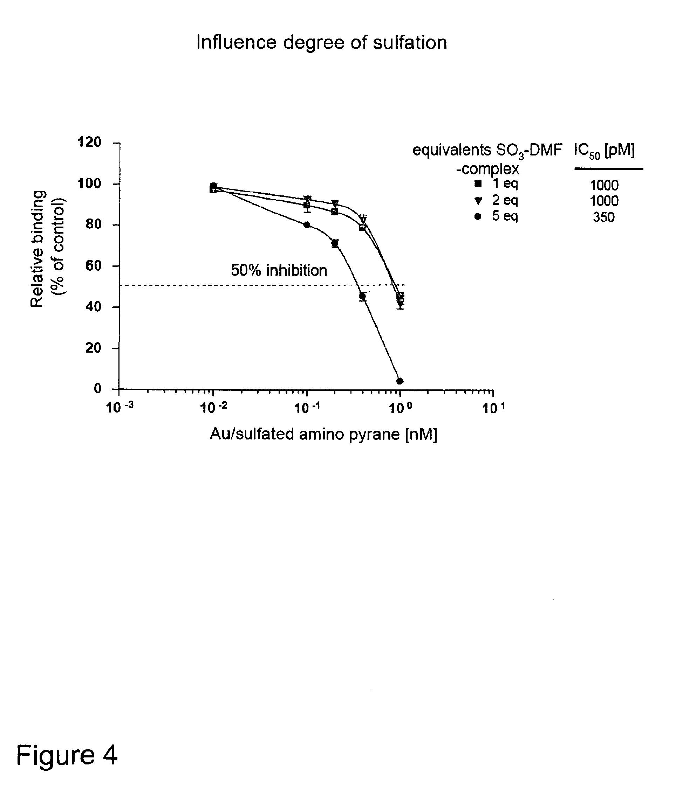

As shown in **Figure 3**, all three nanoparticles inhibited L-selectin-ligand interaction at concentrations of the picomolar range. Gold nanoparticles functionalized with dendritic polyglycerol was used as negative control. The degree of sulfation of amino pyrane (nanoparticles 4, sulf-AP) significantly influenced the half inhibitory concentration (see **Figure 4**). As shown in **Table 1**, P-selectin-ligand interaction was inhibited at even lower concentrations of the nanoparticles 1, 2, and 4.

**Table 1 IC₅₀ values measured in competitive in vitro L-selectin- and P-selectin-ligand binding assay using four differently functionalized nanoparticles**

| | MUDS (1) | MUDSulfate (2) | AP (3) | sulf-AP (4) |
|---|---|---|---|---|
| L-selectin | 100 pM | 150 pM | -* | 350 pM |
| P-selectin | 30 pM | 30 pM | 10 nM | 35 pM |

| | | | | |
|---|---|---|---|---|
| *) AP binds exclusively to P-selectin | | | | |

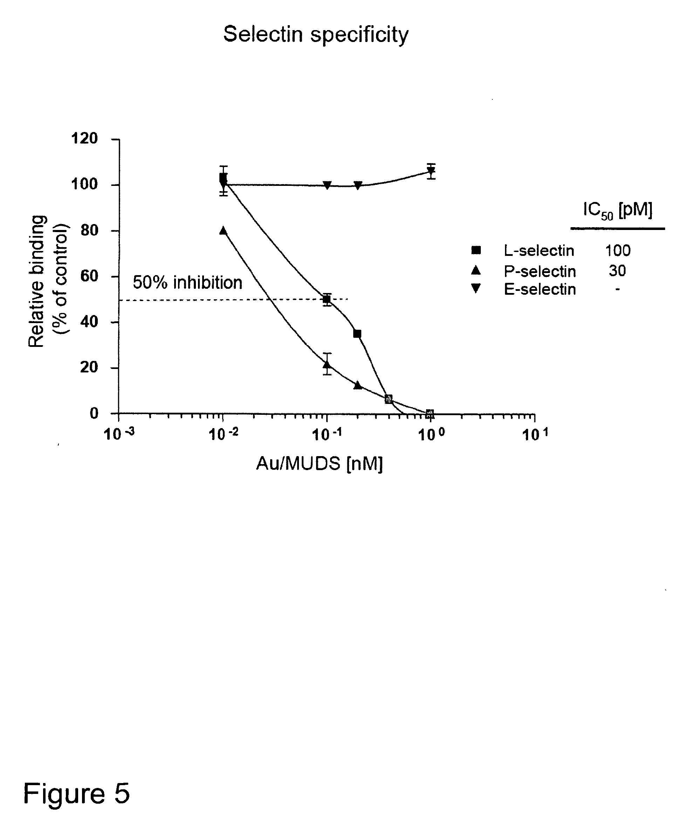

Underlining the specificity of the functionalized nanoparticles according to the present invention, none of the nanoparticles 1, 2, 3, and 4 interacted with E-selectin (for nanoparticle 1, i.e. MUDS see **Figure 5**), and nanoparticle 3 bound exclusively to P-selectin.

## Claims

1. Functionalized nanoparticle, comprising
a core,
a shell coating said core, formed by a monolayer of a linker molecule, and at least one polar functional group covalently linked to said linker molecule.

2. Nanoparticle according to claim 1, wherein said core is formed by gold.

3. Nanoparticle according to claim 1 or 2, wherein said core has a diameter ≤ 50 nm, preferably ≤ 25 nm, more preferably ≤ 15 nm.

4. Nanoparticle according to any of claims 1 to 3, wherein said linker molecule is a linear or branched, preferably a linear alkyl chain, with at least one binding functionality for the attachment of said linker molecule to said core.

5. Nanoparticle according to claim 4, wherein said alkyl chain has 3 to 26 C-atoms, preferably 6 to 26 C-atoms, more preferably 10 to 26 C-atoms.

6. Nanoparticle according to claim 4 or 5, wherein said binding functionality comprises at least one sulfur atom.

7. Nanoparticle according to claim to 6, wherein said binding functionality is selected from the group comprising thiol, thiolate or disulfide.

8. Nanoparticle according to any of claims 1 to 7, wherein said polar functional group is covalently linked to said linker molecule via a bond selected from the group comprising peptide bond, carbon-carbon bond, carbon-oxygen bond, carbon-sulfur bond.

9. Nanoparticle according to any of claims 1 to 8, wherein said polar functional group is selected from the group comprising carboxyl, sulfate, sulfonate, hydroxyl, and a polyhydroxylated heterocyclic compound.

10. Nanoparticle according to any of claim 9, wherein said polar functional group is a polyhydroxylated heterocyclic compound.

11. Nanoparticle according to claim 10, wherein said heterocyclic compound is an oxygen-containing heterocyclic compound.

12. Nanoparticle according to claim 11, wherein said oxygen-containing heterocyclic compound is selected from the group comprising amino pyranes, amino oxepanes, amino oxacanes, and amino furan derivatives.

13. Nanoparticle according to any of claims 10 to 12, wherein said polyhydroxylated heterocyclic compound is oxidized to carboxylic acid in primary hydroxylated positions.

14. Nanoparticle according to any of claims 10 to 13, wherein said polyhydroxylated heterocyclic compound is sulfated.

15. Nanoparticle according to any of the foregoing claims, wherein said linker molecule is represented by the formula 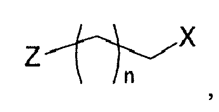 wherein
Z represents a binding functionality as defined in any of claims 4, 6, and 7, wherein Z is selected from the group comprising SH, S⁻, and S₂,
n is not less than 2 and equal to or less than 25, and
X represents a polar functional group as defined in any of claims 1 to 14, wherein
X is selected from the group comprising COOH, OSO₃⁻, SO₃⁻, OH, and a polyhydroxylated heterocyclic compound, preferably an oxygen-containing heterocyclic compound being represented by the formula 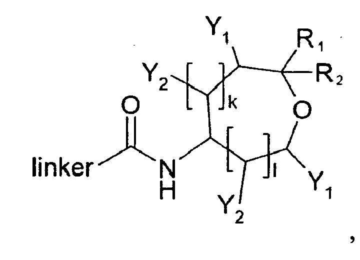 wherein
k is 0 or 1,
1 is 0, 1, or 2,
Y₁ is selected from the group comprising CH₂OH COOH, and CH₂OSO₃⁻,
Y₂ is selected from the group comprising OH and OSO₃⁻,
and R₁ and R₂ are selected from the group comprising H, alkyl, aryl, and heteroaryl, preferably an alkyl, wherein said alkyl is selected from the group comprising C1-C12 alkyl, preferably methyl, ethyl, propyl, isopropyl, butyl, t-butyl, a perhalogenated alkyl, wherein the halogenide is selected from the group comprising F, CI, Br, and I, preferably F, and a hydroxylated alkyl, preferably monohydroxylated alkyl.

16. Method for producing a functionalized nanoparticle according to any of claims 1 to 15, preferably 10 to 15, comprising the covalent linking of polar functional groups, preferably polyhydroxylated heterocyclic compounds as defined in any of claims 1 to 15, preferably 10 to 15, to linker molecules bound to a gold core.

17. Nanoparticle according to any of claims 1 to 15 for the treatment of diseases.

18. Use of a nanoparticle according to any of claims 1 to 15 for the production of a medicament for the treatment of inflammatory diseases.

19. Use according to claim 18, wherein said inflammatory diseases are chronic inflammatory diseases, in particular rheumatoid arthritis, asthma, and psoriasis.

20. Use according to claim 18, wherein said inflammatory diseases are ischemia reperfusion damages or graft repulsion.

21. Use of a nanoparticle according to any of claims 1 to 15 as selectin inhibitor.

22. Use of a nanoparticle according to claim 21 as inhibitor of L-selectin and/or P-selectin and/or E-selectin.
